Europäisches Patentamt

European Patent Office

Office européen des brevets

(19)

(11) Publication number: **0 135 145**

**A2**

(12) # EUROPEAN PATENT APPLICATION

(21) Application number: **84109700.9**

(22) Date of filing: **15.08.84**

(51) Int. Cl.⁴: **B 01 J 23/20**
**C 07 C 29/04**

(30) Priority: **17.08.83 JP 149251/83**

(43) Date of publication of application:
**27.03.85 Bulletin 85/13**

(84) Designated Contracting States:
**AT BE CH DE FR GB IT LI LU NL SE**

(71) Applicant: **CBMM Internacional Ltda.**
**Rua Padre Joao Manoe, 823**
**Sao Paulo Estado de Sao Paulo(BR)**

(72) Inventor: **Kado, Satoshi**
**3-26-1 Miroku-ji**
**Fuhisawa-shi Kanagawa-ken(JP)**

(72) Inventor: **Iizuka, Tokio, Prof.**
**1-7 Nakano-shima Toyohira-ku**
**Sapporo-shi Hokkaido(JP)**

(72) Inventor: **Tanabe, Kozo, Prof.**
**14-11 Sonomachi Oasa**
**Ebetsu-shi Hokkaido(JP)**

(74) Representative: **Patentanwälte Wenzel & Kalkoff**
**Grubes Allee 26 Postfach 730466**
**D-2000 Hamburg 73(DE)**

(54) Solid acid catalysts and process of producing the same.

(57) A solid acid catalyst is obtained by washing niobium hydroxide with water, optionally treating it with sulphuric acid or hydrofluoric acid to obtain hydrated niobium oxide ($Nb_2O_5 \cdot nH_2O$) and then heat-treating the hydrated niobium oxide at a low temperature in the range of 100 to 400 °C, preferably 200 to 300 °C. The solid acid catalyst is catalytically active in olefin hydration reactions and other acid-catalysed reactions involving water, such as hydrolysis, esterification and Beckman transformation reactions.

EP 0 135 145 A2

CBMM Internacional Ltda., Rua Padre Joao Manoe, 923, Sao Paulo, Estado de Sao Paulo, Brazil

Solid Acid Catalysts and Process of Producing the Same

The invention relates to solid acid catalysts and a process of producing such catalysts.

Today's chemical industry uses many processes requiring catalysts which are to a large degree in the form of sulphuric acid and aluminium chloride. These processes have been encountered with various problems, including difficulties lying in the separation and recovery of the catalyst, the formation of byproducts and colourants, the requirement of wastewater treatment, the corrosion of equipment and, when aluminium chloride is used, a large consumption of the catalyst. Therefore, it is desired to replace mineral acids, such as sulphuric or phosphoric acid, with a solid acid catalyst. At present there is no solid acid catalyst that can be used in the presence of water, except for some ion exchange resin catalysts. However, these ion exchange resins are disadvantageous in that they are weak in acid strength, expensive and easy to degrade, thus restricting their scope of use resp. application. A fluoride resin having a higher acid strength than the mentioned ion exchange resins was recently in the news of the industry circles. This resin was found disadvantageous in that it is not yet satisfactory in its acid strength and is very expensive.

It is a main object of the present invention to eliminate these disadvantages and to provide for a solid catalyst having sufficient acid strength as well as for an economical process of manufacturing the same.

In accordance with the present invention this object is achieved by a solid acid catalyst comprising hydrated niobium

oxide ($Nb_2O_5 \cdot nH_2O$) as its essential effective component. Preferably such hydrated niobium oxide is obtained by washing niobium hydroxide with water and then heat-treating the hydrated niobium oxide thus obtained at a low temperature. Expediently the hydrated niobium oxide may be treated with sulphuric acid or hydrofluoric acid prior to the low-temperature heat treatment.

In accordance with the experimental studies it has been found that hydrated niobium oxide pretreated with heat at a moderate temperature in the range of 100 to 400 $^{\circ}$C, preferably 200 to 300 $^{\circ}$C, shows a considerably high acid strength and resistance to poisoning by water. This solid acid catalyst has also been found to be active as the catalyst especially in olefin hydration reactions and likewise highly active in other acid-catalysed reactions involving water, such as hydrolysis, esterification and Beckman transformation.

It has further been found that hydrated niobium oxide shows an improved catalytic activity when it is pretreated with sulphuric acid or hydrofluoric acid prior to the heating at a low temperature.

The following Table 1 reflects the acid strength observed on the surfaces of hydrated niobium oxide ($Nb_2O_5 \cdot nH_2O$) after it has been heat-treated at various temperature levels.

### Table 1

| Pretreatment temperature $^{\circ}$C | 100 | 200 | 300 | 400 | 500 |
|---|---|---|---|---|---|
| Max. acid strength $H_o$ | -5.6 | -8.2 | -8.2 | -5.6 | +1.5 to +3 |

As is apparent from Table 1 the hydrated niobium oxide treated at a temperature of 200 to 300 $^{\circ}$C has an acid strength of $H_O = -8.2$ which corresponds to the acid strength of 90% sulphuric acid. When treated at 100 $^{\circ}$C the compound has an acid strength of $H_O = -5.6$ which corresponds to the acid strength of 71% sulphuric acid. No ordinary metal oxide shows such a high acid strength even when it is heat-treated at a temperature as low as specified herein. Sometimes metal oxides show high acid strengths when they are heat-treated or treated for air removal at a temperature as high as e.g. 500 $^{\circ}$C, but adsorption of water causes their acid strength to drop sharply.

On the contrary, niobium hydroxide shows an acid strength as high as $H_O = -5.6$ when 200 g of niobium hydroxide is suspended in 5 l of distilled water for one or two days, then filtered, washed with water five times and dried at 100 $^{\circ}$C for 40 hours. In the case of other solid acids such as silica-alumina, silica-titania and the like, strongly acidic points appear when they are dehydrated by heat treatment at a high temperature range of 400 to 500 $^{\circ}$C. Niobium hydroxide, on the other hand, develops a high acid strength even under low-temperature treatment such as described above.

In the following, the invention and the advantages achieved thereby will be described in greater detail by way of specific examples and experiments carried out and with reference to the accompanying schematic drawings. In these drawings

Fig. 1    shows the relationship between the air-removing temperature and the catalytic activity in the isomerisation of 1-butene using the catalyst of this invention,

Fig. 2    shows the same relationship as in Fig. 1 in the
          dehydration of 2-butanol, and

Fig. 3    shows the same relationship as in Fig. 1 in the
          polymerisation of propylene.

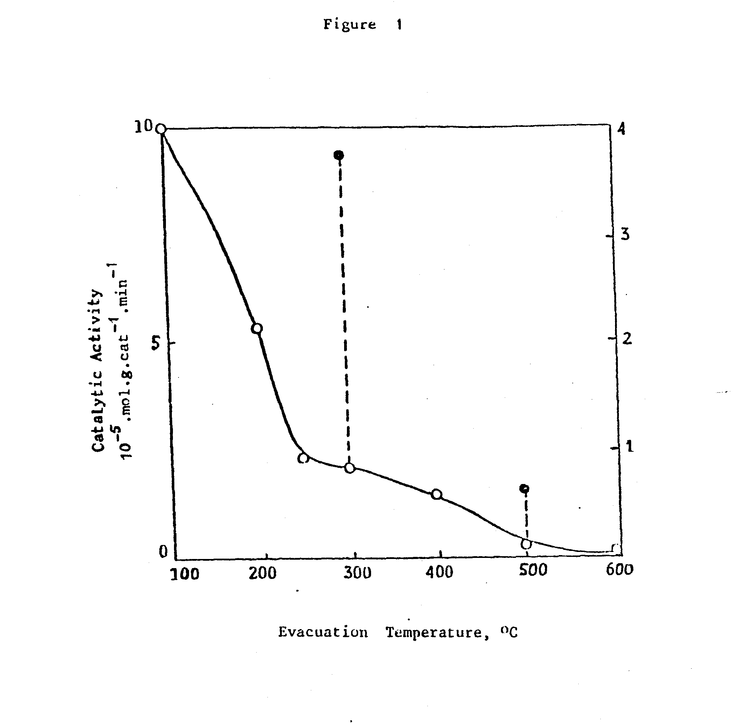

Relating to Fig. 1 the dependence of treatment temperatures on
the catalytic activity of the compound of this invention will
be described, taking isomerisation of 1-butene (conducted at a
reaction temperature of 100 $^{O}$C and a 1-butene pressure of
40 Torr) as the model reaction for the catalyst of this invention.  As becomes apparent from Fig. 1 the catalyst activated
by air removal at 100 $^{O}$C from niobium hydroxide has high
catalytic activity.  The activity drops with increasing temperatures used for air removal.  If, however, the catalyst is
treated for air removal at 300 $^{O}$C, contacted with steam at
normal temperature and air is again removed at 100 $^{O}$C, then
the catalyst returns to almost the same level of activity as
the catalyst simply air-removed at 100 $^{O}$C, as indicated in
Fig. 1 by black circles (●).  In this respect the solid acid
catalyst of hydrated niobium oxide of this invention has a
clear characteristic, considering that conventional solid acid
catalysts are remarkably deactivated by water.

When niobium hydroxide is heated at or above 500 $^{O}$C, crystal-
lisation of niobium oxide proceeds, thereby causing acid
strength to be lost and catalytic activity to decrease.  In the
case of niobium hydroxide obtained from niobium oxalate by
precipitation using acqueous ammonia, any high acid strength
such as is achieved in accordance with the present invention
cannot be obtained if the decomposition of raw materials or the
desorption of ammonia is not sufficient in the low-temperature
pre-treatment.

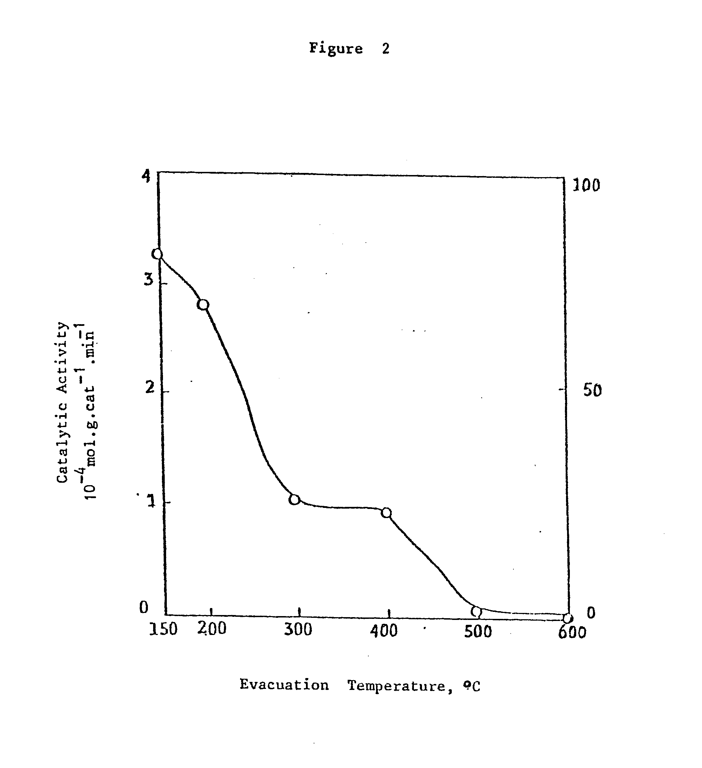

The effect of low-temperature treatment on the catalyst activity can also be demonstrated from the dehydration reaction of
2-butanol (conducted at a reaction temperature of 150 $^{\circ}$C and
a 2-butanol partial pressure of 18 Torr). Fig. 2 indicates the
high activity of the catalyst air-removed at a low temperature
range of 150 to 200 $^{\circ}$C. In addition there is less poisoning
by water formed during the reaction as compared to the use of
other solid acid catalysts.

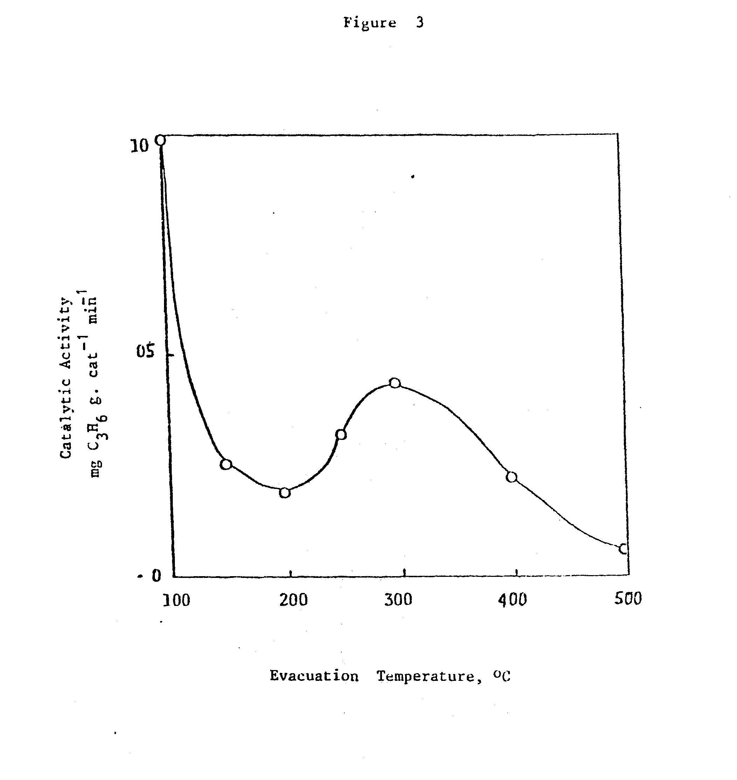

Similar effectiveness of the catalyst according to this
invention can be further demonstrated in the polymerisation of
propylene (conducted at a reaction temperature of 100 $^{\circ}$C and
a propylene pressure of 100 Torr), as can be taken from Fig. 3.

In the following, specific examples will be described.

### Example 1

Three types of catalysts were treated for air removal for two
hours before they were used in the synthesis of ethanol through
hydration of ethylene. These catalysts included a catalyst
obtained by washing niobium hydroxide with water (hereinafter
referred to as $Nb_2O_5$), a second catalyst obtained by dipping niobium hydroxide in an aqueous 1N $H_2SO_4$ solution for
40 minutes followed by filtering and drying (hereinafter refer-
red to as $Nb_2O_5$-$SO_4$), and a third catalyst obtained by
dipping niobium hydroxide in an aqueous 1 wt % HF solution for
two days followed by filtering and drying (hereinafter referred
to as $Nb_2O_5$-F).

A reaction vessel of a closed circulating system was used. A
water vapouriser was fitted to the reaction vessel just
upstream of the vessel. 5 ml of water were introduced into the
vapouriser and the vapouriser was in turn placed in a temperature-controlled bath to supply the system with steam of a constant pressure. The entire reaction system was heated by a cord heater to prevent steam from condensing. The reaction product was allowed to dissolve in water in the vapouriser and a certain amount of the product was taken and analysed by gas chromatography. Reaction conditions included a catalyst in an amount ranging from 0.8 to 0.9 g, an ethylene partial pressure of 400 Torr, a steam partial pressure of 55 Torr and a reaction temperature of 180 $^{O}$C. Table 2 gives the results of this reaction.

Table 2. Hydration Reaction of Ethylene

| Catalyst | Frequency of reaction | Air-removing temperature °C | Ethanol-forming speed ($\times 10^{-2}$ VOL.% $g^{-1}$ $hr^{-1}$) | Maximum acid strength |
|---|---|---|---|---|
| $Nb_2O_5$ | 1st | 200 | 0.05 | -8.2 |
| | 2nd | 200 | 0.11 | - |
| | 3rd | 200 | 0.19 | - |
| | 8th | 200 | 0.38 | - |
| | (Constant activity level was reached.) | | | |
| $Nb_2O_5$-$SO_4$ | 1st | 200 | 0.18 | -8.2 |
| | 2nd | 200 | 0.21 | - |
| | 3rd | 200 | 0.25 | - |
| $Nb_2O_5$-F | 1st | 200 | 0.25 | -8.2 |
| | 2nd | 200 | 0.51 | - |
| | 3rd | 200 | 0.57 | - |
| | 5th | 200 | 0.64 | - |
| | (Constant activity level was reached.) | | | |
| Solid Phosphoric acid | 1st | 180* | 0.87 | - |
| | 2nd | 20** | 0.17 | - |

*) Air-removing treatment for 30 minutes

**) Air-removing treatment for 2 hours.

0135145

The reaction was conducted for three hours in the presence of $Nb_2O_5$. After the used $Nb_2O_5$ was treated for air removal it was used again in repeated reactions. Its activity was found to increase with its repeated use and after eight or nine times of repetition the activity reached a constant level. $Nb_2O_5$-F also showed greatly increased activity. Like $Nb_2O_5$ the activity of $Nb_2O_5$-F increased with repeated use in reactions and reached a constant level after four or five times of repeated use.

Solid phosphoric acid, now commercially used in the hydration of ethylene, was treated under the same reaction conditions as described above. The used phosphoric acid was then treated for air removal for two hours. The results of this test are also included in Table 2 which indicates a remarkable drop in activity. This is probably because phosphoric acid has escaped from the reaction system through evaporation. There is no such fear when the solid acid catalyst according to the present invention is used.


Example 2


Isopropanol was synthesised by means of propylene hydration under pressure using a flowing reaction system. A highly concentrated aqueous solution of isopropanol resulted, as given in Table 3 below.

Table 3. Results of Propylene Hydration Reaction

| Reaction time hr | 1 | 2 | 3 | 4 | 5 | 6 | 7 |
|---|---|---|---|---|---|---|---|
| Volume of isopropanol formed, x $10^{-2}$ cc/g·hr | – | 2.14 | 2.55 | 7.80 | 12.1 | 14.4 | 13.2 |

Reaction temperature:       200 °C

Reaction pressure:          9 Atm

Flow rate of $H_2O$         0.6 cc/hr

Propylene/$H_2O$ ratio:     8

The catalyst activity increased with lapsed time and reached a constant level in six or seven hours.

The compound of this invention shows favourable activity as a catalyst and high resistance to catalyst poisoning, thus promising a high industrial value.

Patent Claims:

1. A solid acid catalyst, c h a r a c t e r i s e d i n
   that it comprises hydrated niobium oxide as its essential
   active component.

2. A solid acid catalyst as claimed in claim 1,
   c h a r a c t e r i s e d i n that the hydrated niobium
   oxide is obtained from niobium hydroxide which is washed
   with water and subsequently heat-treated at a low
   temperature in the range of 100 to 400 $^{o}$C, preferably 200
   to 300 $^{o}$C.

3. A solid acid catalyst as claimed in claim 2,
   c h a r a c t e r i s e d i n that the hydrated niobium
   oxide has been treated with sulphuric acid subsequent to
   said washing and prior to said heat-treatment.

4. A solid acid catalyst as claimed in claim 2,
   c h a r a c t e r i s e d i n that the hydrated niobium
   oxide has been treated with hydrofluoric acid subsequent to
   said washing and prior to said heat-treatment.

5. A process of producing a solid acid catalyst,
   c h a r a c t e r i s e d by the steps of
   a) washing niobium hydroxide with water to obtain hydrated
      niobium oxide and
   b) subsequently heat-treating said hydrated niobium oxide
      at a low temperature in the range of 100 to 400 $^{o}$C,
      preferably 200 to 300 $^{o}$C.

6. A process of producing a solid acid catalyst,
   c h a r a c t e r i s e d by the steps of
   a) washing niobium hydroxide with water,

b) treating the hydrated niobium oxide thus obtained with sulphuric acid and

c) subsequently heat-treating said hydrated niobium oxide at a low temperature in the range of 100 to 400 $^{o}C$, preferably 200 to 300 $^{o}C$.

7. A process of producing a solid acid catalyst, c h a r a c t e r i s e d  b y  the steps of
   a) washing niobium hydroxide with water,
   b) treating the hydrated niobium oxide thus obtained with hydrofluoric acid and
   c) subsequently heat-treating said hydrated niobium oxide at a low temperature in the range of 100 to 400 $^{o}C$, preferably 200 to 300 $^{o}C$.

8. A process for preparing alcohol by hydration of an olefin using the solid acid catalyst as claimed in any one of claims 1 to 4, c h a r a c t e r i s e d  i n  that olefin is reacted with water in the presence of a catalytically effective amount of hydrated niobium oxide.

9. A process as claimed in claim 8, c h a r a c t e r i s e d i n  that the olefin is ethylene and the alcohol is ethanol.

10. A process as claimed in claim 8, c h a r a c t e r i s e d i n  that the olefin is propylene and the alcohol is isopropanol.

Figure 1

0135145

Figure 2

Evacuation  Temperature, °C

0135145

Figure 3

Evacuation Temperature, °C